# EUROPEAN PATENT APPLICATION

(11) **EP 4 361 642 A1**
(43) Date of publication of application: **01.05.2024**
(21) Application number: 23201189.0
(22) Date of filing: 02.10.2023
(51) Int. Cl.: G01N 35/00, B01L 99/00

(54) **ANALYSIS DEVICE, METHOD FOR RESTRICTING FUNCTION, AND NON-TRANSITORY STORAGE MEDIUM**

(30) Priority: 26.10.2022 JP 2022171784
(71) Applicant: ARKRAY, Inc., Kyoto-shi, Kyoto 601-8045 (JP)
(72) Inventor: Kubo, Kosuke, Kyoto-shi, Kyoto, 602-008 (JP)
(74) Representative: Hoffmann Eitle

(57) **Abstract**

An analysis device includes: a main body having a measurement unit that measures a component in a sample; an operation unit that is attachable to and detachable from the main body, and a function restricting unit that restricts a function of at least one of the main body or the operation unit depending on two or more levels of distance between the main body and the operation unit.

## Description

### BACKGROUND

### Technical Field

The present disclosure relates to an analysis device, a method for restricting a function, and a non-transitory storage medium.

For example, Japanese Patent Application Laid-Open (JP-A) No. 2016-151950 describes a printing system including a host computer, a mobile terminal, a server, and a printing device. The server includes storage means that stores a print job received from the host computer, transmission means that transmits identification information of the printing device which will print print data of the print job to the mobile terminal based on identification information of the mobile terminal which is included in the print job, and print control means that transmits the print data stored in the storage means to the printing device in response to a print instruction from the mobile terminal and causes the printing device to print the print data. The mobile terminal includes acquisition means that acquires a distance to the printing device by short-range wireless communication based on the identification information of the printing device received from the server, and transmission means that transmits a print instruction for the print data to the server when the distance acquired by the acquisition means is shorter than a predetermined distance.

In addition, International Publication No. WO2014/132873 describes a control system including a first transmitter, a second transmitter, and a reception device that controls the selfsame device on the basis of radio waves transmitted from the first transmitter and the second transmitter. The first transmitter is provided with a transmission unit that transmits radio waves to the reception device, and the second transmitter is provided with a transmission unit that transmits radio waves to the reception device. The reception device is provided with: a reception unit that receives radio waves transmitted from the first transmitter and the second transmitter; an identification unit that, on the basis of the radio waves received by the reception unit, identifies whether the transmission source of the radio waves is the first transmitter or the second transmitter; a measurement unit that measures the distance between the selfsame device and the transmission source of the radio waves identified by the identification unit; and a control unit that, on the basis of the distance between the selfsame device and the transmission source of the radio waves as measured by the measurement unit, performs first control for imposing a restriction on the selfsame device or second control that does not impose a restriction on the selfsame device.

Incidentally, there are analysis devices to which an operation unit such as a tablet terminal can be detachably attached. In this kind of analysis device, in order to browse measurement results on the operation unit, the operation unit may be brought to a place away from the analysis device to browse the measurement result. In a case in which the operation unit is a certain distance or more away from the analysis device, there is a possibility that analysis processing will not be able to be appropriately executed, and it is conceivable to limit all functions of the device as in JP-A No. 2016-151950 and WO2014/132873.

However, the analysis device has various functions such as a function of managing various types of information (analysis information, sample information, measurement results, or the like) or a function related to maintenance of the device, in addition to analysis. In this kind of analysis device, if all functions of the device are restricted in the same manner, user convenience deteriorates. For example, a user cannot continue measurement or cannot access various types of information.

For example, in a case in which a sample is a biological sample, there is a possibility that the biological sample may fall over, or a person around the device may come into contact with a device drive unit to which the biological sample is attached if the device is driven without checking a device status. Since the amount of a biological sample that can be collected may be limited, losing the sample due to falling over of the sample is a serious risk. In addition, there is a risk that an infectious disease or the like may be transmitted from a biological sample to another person, and thus, care needs to be taken in handling the biological sample.

On the other hand, in a medical site or the like, since there is a demand for browsing measurement results at an early stage, the user convenience deteriorates if all functions of the device are restricted in the same manner. For example, the user cannot continue the measurement or cannot access various types of information.

### SUMMARY

The present disclosure has been made in view of the above-described points and provides an analysis device, a method for restricting a function, and a non-transitory storage medium by which a function of a device can be restricted depending on a distance between an analysis device main body and an operation unit that is attachable to and detachable from the main body, while a decrease in user convenience is suppressed.

An analysis device according to an aspect of the present disclosure includes a main body having a measurement unit that measures a component in a sample and an operation unit that is attachable to and detachable from the main body, the analysis device including: a function restricting unit that restricts a function of at least one of the main body or the operation unit depending on two or more levels of distance between the main body and the operation unit.

According to the present disclosure, it is possible to restrict a function of a device depending on a distance between an analysis device main body and an operation unit that is attachable to and detachable from the main body, while a decrease in user convenience is suppressed.

### BRIEF DESCRIPTION OF THE DRAWINGS

Fig. 1 is a perspective view schematically illustrating an example of an analysis device according to a first embodiment.
Fig. 2 is a block diagram illustrating an example of a configuration of the analysis device according to the first embodiment.
Fig. 3 is a view schematically illustrating an example of a configuration of a main part of the analysis device according to the first embodiment.
Fig. 4 is a perspective view illustrating an example of a configuration of a main part of a first optical measurement unit according to the first embodiment.
Fig. 5 is a cross-sectional view illustrating a cross section of V-V illustrated in Fig. 3.
Fig. 6 is a block diagram illustrating an example of a configuration of an operation unit according to the first embodiment.
Fig. 7 is a block diagram illustrating an example of a functional configuration of a control unit according to the first embodiment.
Fig. 8 is a diagram for describing a functional restriction depending on a distance between a main body and the operation unit according to the first embodiment.
Fig. 9 is a front view illustrating an example of a functional restriction notifying screen according to the first embodiment.
Fig. 10 is a flowchart illustrating an example of a flow of a function restricting process executed by a control program of the analysis device according to the first embodiment.
Fig. 11 is a diagram for explaining a functional restriction depending on a distance between a main body and an operation unit according to a second embodiment.
Fig. 12 is a flowchart illustrating an example of a flow of a function restricting process executed by a control program of an analysis device according to the second embodiment.
Fig. 13 is a diagram for describing a functional restriction in a case in which the operation unit according to a third embodiment supports a plurality of analysis devices.
Fig. 14 is a front view illustrating an example of a device identifying screen according to the third embodiment.

### DETAILED DESCRIPTION

Hereinafter, an example of a mode for carrying out a technology of the disclosure will be described in detail with reference to the drawings. Moreover, configurational elements and processes having the same operation, effect, and function are denoted by the same reference numerals throughout the drawings, and redundant description thereof may be omitted as appropriate. The drawings are only schematically illustrated to the extent that the technology of the disclosure can be sufficiently understood. Therefore, the technology of the disclosure is not limited only to the illustrated example. In addition, in this embodiment, description of a configuration that is not directly related to the disclosure or a well-known configuration may be omitted.

### [First Embodiment]

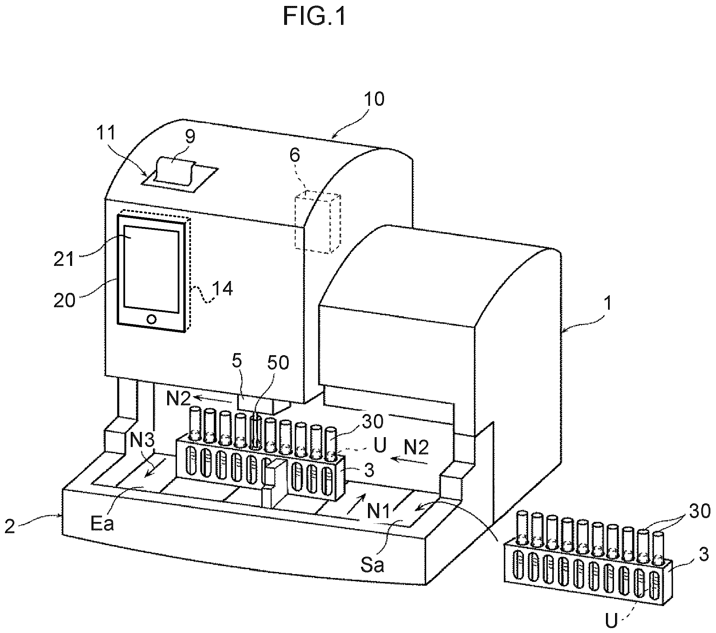

First, a specific configurational example of an analysis device according to a first embodiment will be described with reference to Figs. 1 to 5. Moreover, in the following description, an analysis device that analyzes a coloring state of a test paper will be described as an example; however, an analysis device using liquid chromatography or an analysis device that images a component to be analyzed and performs image analysis may be used. Examples of samples to be analyzed include water and a biological sample (urine, blood, body fluids, etc.). Examples of components to be analyzed in a sample include glycohemoglobin in blood, white blood cells in urine, red blood cells, epithelial cells, and the like.

Fig. 1 is a perspective view schematically illustrating an example of an analysis device 10 according to the first embodiment.

As illustrated in Fig. 1, the analysis device 10 illustrated in the embodiment integrally includes a main body 1, a conveyance device 2, and an operation unit 20. The analysis device 10 is, for example, for performing analysis processing of urine U contained in a container 30 and has a configuration in which the conveyance device 2 is assembled at a front surface portion of the main body 1.

Moreover, in a case in which urine U is used as a sample, a test paper to be adopted is, for example, a urine test paper having a reaction region for a large number of test items for testing test items such as urine proteins, urinary ketone bodies, or urine sugars. The sample is not limited to the urine U and may be a biological sample other than the urine U (for example, blood, plasma, saliva, or the like). In addition, the sample may be a liquid for water quality inspection.

The conveyance device 2 is a device for conveying a rack 3 that holds the container 30 upright in a fixed path. The conveyance device 2 can have a configuration similar to that of a conventionally known conveyance device (for example, a conveyance device described in Japanese Patent Application Laid-Open (JP-A) No. 2009-229233), and the description of details of a specific structure thereof will be omitted. In the conveyance device 2, when the rack 3 is loaded into a predetermined starting region Sa, the rack 3 is sequentially conveyed in directions denoted by arrows N1 to N3 and finally reaches a predetermined end region Ea. In a process in which the rack 3 is conveyed in the direction of the arrow N2, an operation of collecting the urine U from the container 30 is performed by a suction nozzle 50 to be described below.

The operation unit 20 includes a display 21. The operation unit 20 displays an operation screen of the analysis device 10 and receives an operation input from a user via the operation screen. The operation unit 20 is detachably connected to the main body 1 via an attachment and detachment mechanism 14.

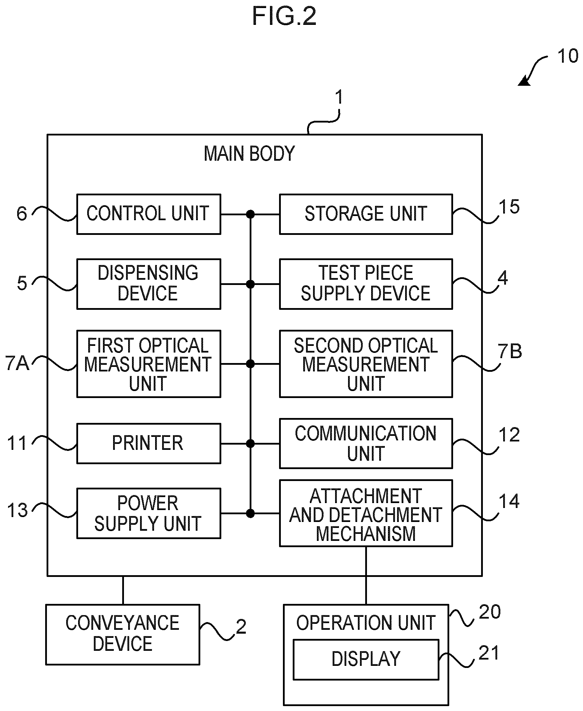

Fig. 2 is a block diagram illustrating an example of a configuration of the analysis device 10 according to the first embodiment.

As illustrated in Fig. 2, the main body 1 of the analysis device 10 according to the embodiment includes a test piece supply device 4, a dispensing device 5, a control unit 6, a first optical measurement unit 7A, a second optical measurement unit 7B, a printer 11, a communication unit 12, a power supply unit 13, the attachment and detachment mechanism 14, and a storage unit 15.

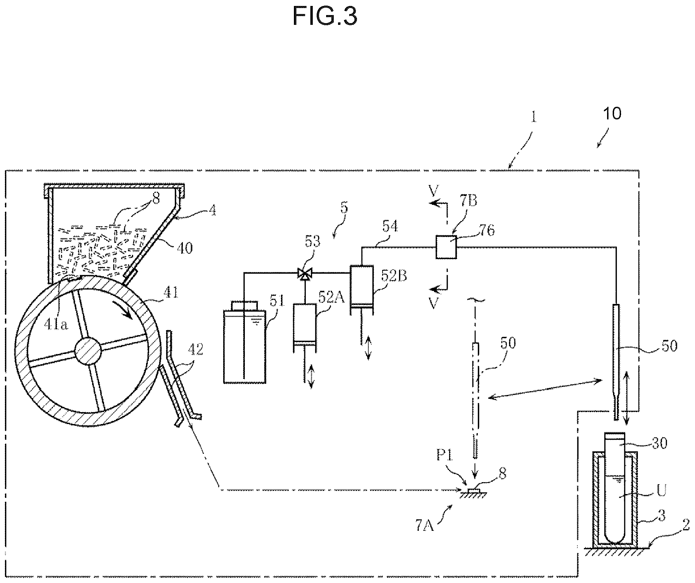

Fig. 3 is a view schematically illustrating an example of a configuration of a main part of the analysis device 10 according to the first embodiment.

As illustrated in Fig. 3, the test piece supply device 4 is a device for supplying a test paper 8 for urine analysis to a predetermined position P1 of the first optical measurement unit 7A. The test piece supply device 4 includes a hopper 40 that accommodates a plurality of test papers 8 and a rotary drum 41 for taking out the test papers 8 one by one from the hopper 40. The rotary drum 41 has a recess portion 41a in which only one test paper 8 can be fitted, on an outer circumferential surface of the rotary drum, and rotation of the rotary drum 41 causes the test paper 8 fitted in the recess portion 41a to be transferred to an outside of the hopper 40 and loaded in a pair of guides 42. Thereafter, the test paper 8 is transferred to the predetermined position P1 by a transfer device (not illustrated).

The dispensing device 5 can perform an operation of collecting the urine U from the container 30 using the suction nozzle 50 and dispensing (spotting) the collected urine U on the test paper 8. The suction nozzle 50 is vertically and horizontally movable by a drive mechanism (not illustrated). The dispensing device 5 has a function of cleaning the suction nozzle 50 and includes a cleaning liquid tank 51 storing a cleaning liquid such as distilled water, syringe pumps 52A and 52B, a direction switching valve 53 such as a three-way valve, and a flow channel 54 formed in series from the cleaning liquid tank 51 to the suction nozzle 50. The flow channel 54 is configured of an appropriate tube. Operations of the syringe pumps 52Aand 52B enable a negative pressure for sucking the urine U and a positive pressure for discharging the urine U to be generated in the suction nozzle 50. In addition, after the discharge of the urine U is finished, the cleaning liquid in the cleaning liquid tank 51 can be sent into the suction nozzle 50 to clean the suction nozzle. Such a configuration is similar to, for example, a dispensing device described in Japanese Patent Application Laid-Open (JP-A) No. 2000-321270, and description of details thereof will be omitted. Moreover, in the embodiment, the second optical measurement unit 7B for a urine color test is provided at a position on the flow channel 54 of the dispensing device 5, which will be to be described below.

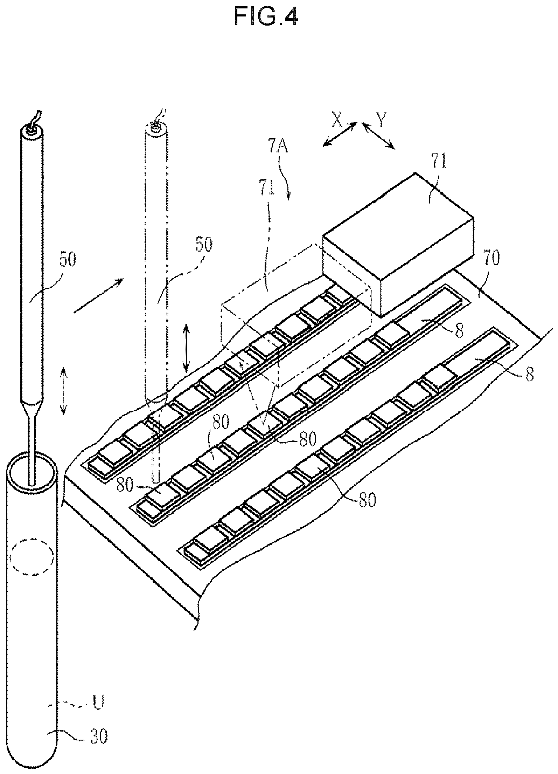

Fig. 4 is a perspective view illustrating an example of a configuration of a main part of the first optical measurement unit 7A according to the first embodiment.

As illustrated in Fig. 4, the first optical measurement unit 7A for detecting a color reaction includes a placement table 70 for placing the plurality of test papers 8 and an optical measuring instrument 71. Each test paper 8 has a plurality of reagent pads 80, and the urine U collected by the suction nozzle 50 is dispensed onto the reagent pads 80. Each of the plurality of reagent pads 80 contains a reagent that reacts with a predetermined component in the urine U and develops color to a degree corresponding to the concentration of the component. As the reagent, reagents of various components corresponding to test items of the urine U are used. The optical measuring instrument 71 is movable in an X direction and a Y direction, irradiates each reagent pad 80 with light having a predetermined wavelength range from a light source (not illustrated) after the urine U has been spotted, and receives reflected light by a light receiving element (not illustrated). The light reflectance of a reagent can be measured based on the amount of light received by the light receiving element. This light reflectance corresponds to a degree of color reaction (degree of color development) between a specific component of the urine U and the reagent, and presence or absence or concentration (including a semi-quantitative value) of the specific component in the urine is determined based on the value of the light reflectance. This determination may be performed by, for example, the control unit 6 or the first optical measurement unit 7A.

Moreover, the first optical measurement unit 7A is an example of a measurement unit and is a measurement unit for detecting a color reaction that measures a coloring state of the test paper 8 under a certain condition. Examples of the certain condition here include conditions that the amount of light with which the test paper 8 is irradiated is constant, that the test paper 8 is irradiated with uniform light, that a distance between the test paper 8 and the light source is constant, and that light having a wavelength suitable for measurement is used in a case of a configuration for reading reflection of light. Moreover, the analysis of the coloring state is not limited to an optical technique using the first optical measurement unit 7A, and the coloring state of the test paper 8 may be analyzed from a captured image obtained by imaging the coloring state of the test paper 8 by an imaging unit (not illustrated) such as a camera.

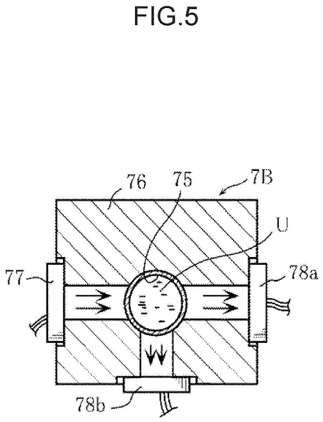

On the other hand, as illustrated in Fig. 3, the second optical measurement unit 7B for the urine color test is provided at a position on the flow channel 54 and can allow the urine U sucked by the suction nozzle 50 to flow into a cell 75 illustrated in Fig. 5 of the second optical measurement unit 7B.

Fig. 5 is a cross-sectional view illustrating a cross section of V-V illustrated in Fig. 3.

As illustrated in Fig. 5, the second optical measurement unit 7B includes the transparent cylindrical cell 75 into which the urine U flows, a light source 77 attached to a light shielding block 76 surrounding the cell 75, and two light receiving elements 78a and 78b. The light source 77 can irradiate the cell 75 with a plurality of types of light having different wavelengths. The light receiving element 78a receives light transmitted through the urine U in the cell 75, and the absorbance of the urine U per predetermined optical path length is obtained based on the amount of light received by the light receiving element 78a. This absorbance is the absorbance of the urine U itself that is not reacted with the reagent. The light receiving element 78b is for receiving light scattered and reflected from the urine U and can determine the turbidity of the urine U based on the amount of light received by the light receiving element 78b.

In Fig. 2 described above, the printer 11 prints and outputs analysis results of the urine U and data of other predetermined items on a predetermined sheet 9.

The control unit 6 includes, for example, a processor such as a central processing unit (CPU) and executes operation control of each unit of the analysis device 10 and various types of data processing in accordance with a control program stored in the storage unit 15. This control program is an example of a function restriction program.

The storage unit 15 can be accessed by the control unit 6 and stores operation control of each unit of the analysis device 10, a control program for executing various types of data processing, various types of data, and the like. Moreover, the storage unit 15 may be integrally provided in the control unit 6.

The communication unit 12 is a communication interface for performing wireless communication such as a wireless local area network (LAN) or short-range wireless communication such as near field communication (NFC) and can perform data communication with the operation unit 20 away from the main body 1 by wireless communication.

The power supply unit 13 is a power supply for supplying electric power to the analysis device 10 and is, for example, an AC power supply, a battery, or the like.

The attachment and detachment mechanism 14 enables the operation unit 20 to be attached and detached. The interface may be an interface for electrically connecting the operation unit 20 and the main body 1. The attachment and detachment mechanism 14 may have any structure in which the operation unit 20 can be attached and detached, and the structure is not particularly limited.

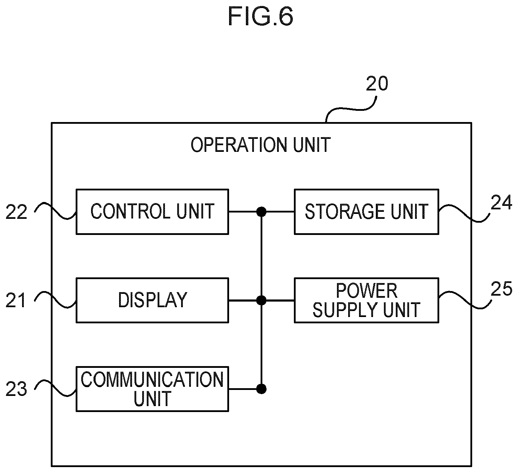

Fig. 6 is a block diagram illustrating an example of a configuration of the operation unit 20 according to the first embodiment. For example, a portable information processing device such as a tablet terminal, a smartphone, or a notebook personal computer is applied to the operation unit 20.

As illustrated in Fig. 6, the operation unit 20 according to the embodiment includes the display 21, a control unit 22, a communication unit 23, a storage unit 24, and a power supply unit 25.

The display 21 includes a display screen such as a liquid crystal display panel and performs screen display for guiding an operation of the analysis device 10, for example. Moreover, the analysis results of the urine U may be displayed on the display 21. The display 21 integrally includes, for example, a touch panel.

The control unit 22 includes, for example, a processor such as a CPU and executes operation control of each unit of the operation unit 20 and various types of data processing in accordance with a control program stored in the storage unit 24.

The communication unit 23 is a communication interface for performing wireless communication such as wireless LAN or short-range wireless communication such as NFC and can perform data communication with the main body 1 away from the operation unit 20 by wireless communication.

The storage unit 24 can be accessed by the control unit 22 and stores operation control of each unit of the operation unit 20, a control program for executing various types of data processing, or various types of data. Moreover, the storage unit 24 may be integrally provided in the control unit 22.

The power supply unit 25 is a power supply for supplying electric power to the operation unit 20 and is, for example, a battery or the like. The power supply unit 25 is a power supply independent of the power supply unit 13 on the main body 1 side and can supply power to the operation unit 20 even when the main body 1 is turned off. In addition, in a case in which the operation unit 20 is mounted by the attachment and detachment mechanism 14 of the main body 1 and power can be supplied from the main body 1, the power supply from the power supply unit 25 is automatically stopped, and the power supply is controlled to be performed from the power supply unit 13 on the main body 1 side. In this case, the power supply unit 25 is charged with the power supplied from the power supply unit 13 on the main body 1 side.

Incidentally, as described above, in a case in which the operation unit 20 is away from the main body 1 by a certain distance or more, there is a possibility that the analysis processing cannot be appropriately executed, and it is conceivable to limit all the functions of the analysis device 10. However, the analysis device 10 has various functions such as a function of managing various types of information (analysis information, sample information, measurement results, or the like) or a function related to maintenance of the device, in addition to the analysis. In this kind of analysis device 10, if all functions of the device are restricted at the same manner, user convenience deteriorates. For example, a user cannot continue measurement or cannot access various types of information.

In contrast, the analysis device 10 according to the embodiment includes a function restricting unit that restricts a function of at least one of the main body 1 or the operation unit 20 depending on two or more levels of distance (hereinafter, simply referred to as a "distance") between the main body 1 and the operation unit 20.

For example, the analysis device 10 according to the embodiment can be continuously used by the user while providing functional restriction to satisfy a request for continuously measuring a sample as much as possible on the basis of a management request of an examination room or a request for browsing a measurement result immediately after measurement.

Hereinafter, some examples of viewpoints of a relationship between the functional restriction and the distance will be described. The "distance" mentioned herein is a distance between the main body 1 and the operation unit 20.

### (1) Example of Viewpoint of Safety Aspect during Device Drive

A distance at which whether the analysis device 10 can be safely driven can be checked is, for example, less than 3 m. If the analysis device 10 is driven in a situation where it cannot be checked that there is a person around the analysis device 10, there is a possibility that a person around the analysis device 10 may be injured by being caught in the drive of the device or the like. The situation where there is a person around the analysis device 10 includes a situation or the like in which another user is touching the device for maintenance of the analysis device 10. Therefore, it is desirable to limit the driving of the analysis device 10 in a case in which the distance exceeds a distance at which whether the analysis device 10 can be safely driven can be checked. Moreover, whether or not the distance is a distance at which a measure can be taken when an error occurs in the device may be used as a viewpoint.

### (2) Example of Viewpoint of Safe Handling of Sample

The distance at which whether the sample is appropriately set in the device can be visually recognized is, for example, less than 1 m. If a tube or the like containing the sample is not appropriately installed in the device, the tube or the like may fall over as the device is driven, and the sample may be lost. In addition, in particular, in a case in which a biological sample is handled as an analysis target, if the tube or the like containing the biological sample falls over, the biological sample is directly touched by the user, and a risk of infection derived from the biological sample occurs. Further, it is necessary to acquire the biological sample again, and an excessive burden is imposed on a biological sample supplier (for example, a patient). Therefore, it is desirable to limit use of a drive system that handles the sample when a distance exceeds the distance at which whether the sample is appropriately set in the device can be visually recognized.

### (3) Example of Viewpoint of Information Management

Taking out of information on the sample measured by the analysis device 10 or an obtained result to the outside leads to information leakage. In particular, in a case in which the sample is a biological sample, it is a serious problem that information such as personal information and medical history of a biological sample supplier and information regarding the measurement result are taken out to the outside. Therefore, in a case in which the operation unit 20 is taken out from a room where the main body 1 is installed, it is desirable to restrict access to the information by the operation unit 20.

Moreover, the distance between the main body 1 and the operation unit 20 may be identified on the basis of the signal strength when communication is performed between the main body 1 and the operation unit 20. In addition, the distance may be identified by identifying access points of the wireless LAN of the main body 1 and the operation unit 20, respectively, or the distance may be estimated using the global positioning system (GPS) in the case of outdoors. Furthermore, the distance may be identified on the basis of information (for example, a location of the user) input from the user. Moreover, the distance here need not be an accurate distance between the main body 1 and the operation unit 20 and may be an estimated distance.

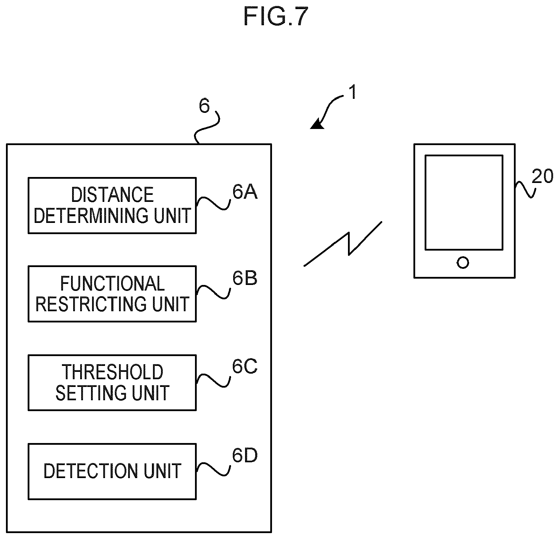

Specifically, the control unit 6 of the main body 1 according to the embodiment functions as units illustrated in Fig. 7 by executing control programs stored in the storage unit 15.

Fig. 7 is a block diagram illustrating an example of a functional configuration of the control unit 6 according to the first embodiment.

As illustrated in Fig. 7, the control unit 6 according to the embodiment functions as a distance determining unit 6A, a function restricting unit 6B, a threshold setting unit 6C, and a detection unit 6D by executing the control programs stored in the storage unit 15. Moreover, the distance determining unit 6A, the function restricting unit 6B, the threshold setting unit 6C, and the detection unit 6D may be implemented by the control unit 22 of the operation unit 20 executing control programs stored in the storage unit 24.

For example, the distance determining unit 6A determines the distance between the main body 1 and the operation unit 20 on the basis of the signal strength when communication is performed between the main body 1 and the operation unit 20. As a type of communication, for example, wireless communication such as wireless LAN or short-range wireless communication such as NFC is applied, and more specifically, for example, Wi-Fi (registered trademark), Bluetooth (registered trademark), radio frequency identification (RFID), or the like can be applied. Moreover, determination of the distance is not limited to the determination on the basis of the signal strength, and for example, when the user is outdoors, the distance may be determined using the GPS, or the distance may be determined by identifying the access point of a wireless LAN.

The function restricting unit 6B restricts the function of at least one of the main body 1 or the operation unit 20 depending on two or more levels of distance determined by the distance determining unit 6A. Examples of a technique of restricting the function include changing an instruction that can be received by the main body 1 from the operation unit 20 (that is, restricting the function of the device by rejecting a specific instruction), disabling a button related to a specific instruction of the operation unit 20 to the main body 1 (for example, grayed-out display of a button, non-display of a button, disablement of a button, and the like), returning the operation unit 20 to a login screen and disabling buttons related to all instructions to the main body 1 in the case of a long distance, or the like. Moreover, in a case in which the distance determining unit 6A determines the distance between the main body 1 and the operation unit 20 on the basis of the signal strength, the function restricting unit 6B may restrict at least one function of the main body 1 or the operation unit 20 according to the signal strength.

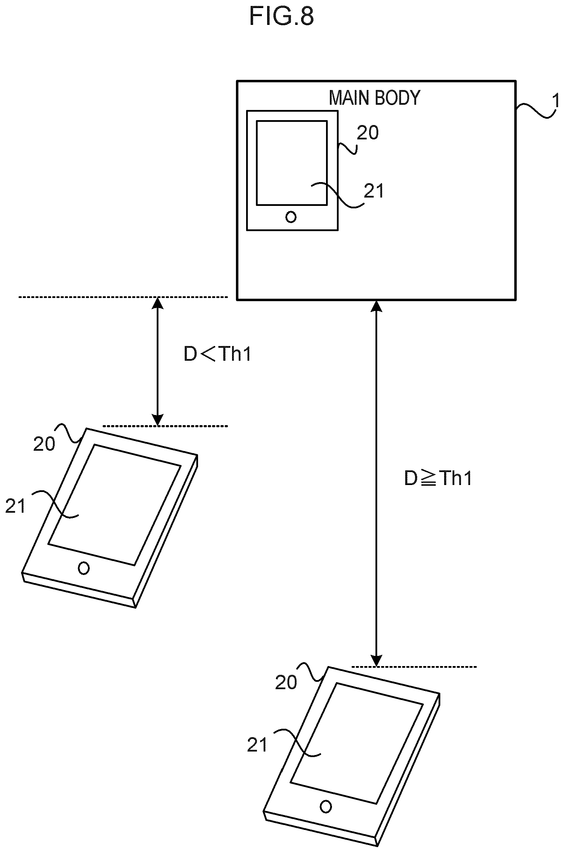

Fig. 8 is a diagram for explaining a functional restriction depending on a distance D between the main body 1 and the operation unit 20 according to the first embodiment.

As illustrated in Fig. 8, in a case in which the operation unit 20 is attached to the main body 1, the function restricting unit 6B according to the embodiment permits the use of all the functions. In a case in which the operation unit 20 is detached from the main body 1 and the distance D is less than a threshold Th1, the function restricting unit 6B restricts at least some functions of the drive system. In a case in which the distance D is equal to or more than the threshold Th1 (for example, outside an examination room, outside a facility, or the like), the function restricting unit 6B restricts the use of all the functions. That is, the function of at least one of the main body 1 or the operation unit 20 is restricted at a first level in a case in which the operation unit 20 is detached from the main body 1 and the distance D is less than the threshold Th1 and at a second level in a case in which the distance D is equal to or more than the threshold Th1.

Specifically, in a case in which the operation unit 20 is detached from the main body 1 and the distance D is less than the threshold Th1, at least some functions of the drive systems are limited, and the functions that do not involve driving are permitted. The limited function is a function that is triggered by a user's operation and is accompanied by an operation in a driving section of the analysis device 10 and includes, for example, a start of analysis, a start of a maintenance operation, a start of a calibration operation, a start of an instrument adjustment/test, a start of replacement of consumables, an operation for troubleshooting an error/trouble, a start of a shutdown operation, or the like. On the other hand, the permitted functions include, for example, browsing of various types of information on the operation unit 20, changing of the setting of the analysis device 10 (enabling access to information related to the analysis device 10), or the like. By appropriately limiting the function of the device in this manner, for example, risks such as a case in which a sample rack falls over and the device is submerged or broken, a case in which a surrounding person is infected by exposure of the sample, a case in which the nozzle is stuck to a surrounding person and the surrounding person is injured, and the like are avoided.

In addition, in a case in which the distance D is equal to or more than the threshold Th1, for example, in a case in which the operation unit 20 is to be taken out of the examination room or out of the facility, the use of all the functions is restricted. That is, since access from the operation unit 20 to various kinds of information stored in the main body 1 is restricted, leakage of information to the outside is prevented.

Moreover, examples of the "drive system" include a drive system necessary for measurement of components in a sample and a drive system necessary for maintenance without using a sample. In a case in which the operation unit 20 is detached from the main body 1 and the distance is less than the threshold, the function restricting unit 6B may restrict at least some functions of the drive system necessary for measuring the component in the sample. In this case, since the function of the drive system necessary for maintenance is permitted, the maintenance operation is enabled.

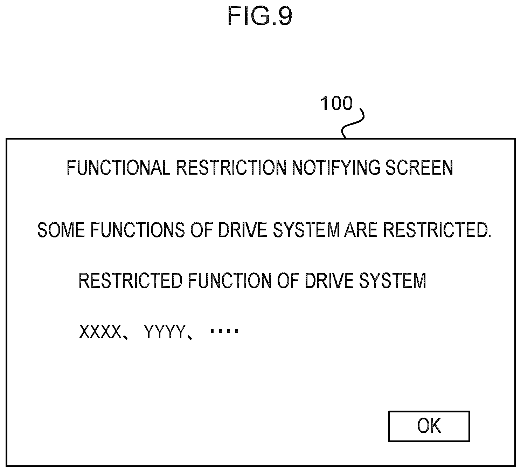

Fig. 9 is a front view illustrating an example of a functional restriction notifying screen 100 according to the first embodiment.

As an example, the functional restriction notifying screen 100 illustrated in Fig. 9 is a screen displayed on the operation unit 20 in a case in which some functions of the drive system are restricted. Consequently, the user of the operation unit 20 can understand which function is restricted.

Returning back to Fig. 7, the threshold setting unit 6C displays a setting screen on the operation unit 20 and enables the threshold Th1 to be set according to a use environment of the user. For example, in a case in which the user who operates the operation unit 20 cannot see the main body 1 although the distance to the main body 1 is short, the threshold Th1 can be set so that all functions are limited even with a short distance.

The detection unit 6D detects a person around the main body 1. The detection unit 6D detects whether or not there is a person around the main body 1 by using, for example, a camera, a human sensor (not illustrated), or the like provided in the main body 1. In a case in which no person is detected around the main body 1 by the detection unit 6D, the function restricting unit 6B may change the function to be restricted. Specifically, if there is no person around the main body 1, for example, a risk of infection is considered to be small even if the sample is exposed at the time of measurement. Therefore, a change may be performed to permit all functions regardless of the distance.

In addition, the function restricting unit 6B may change the function to be restricted on the basis of identification information of the user who logs in the analysis device 10. Examples of a login method into the analysis device 10 include input of identification information of the user to the operation unit 20, reading of identification information of the user by the analysis device 10 or the operation unit 20, and the like. As the identification information of the user, for example, a user identification (ID) is used. In addition, reading the identification information of the user includes reading the user ID described in the ID card of the user. An attribute of the user is associated with the user ID, and it is possible to determine whether the user is, for example, an administrator or a measurement operator by the user ID. Specifically, for example, in the case of an administrator, all functions may be permitted, and in the case of a measurement operator, some of the functions may be restricted.

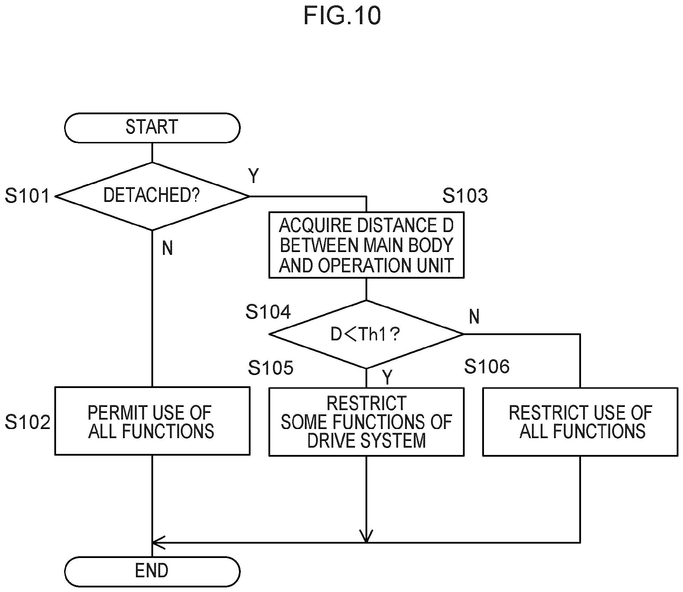

Next, the operation of the analysis device 10 according to the first embodiment will be described with reference to Fig. 10.

Fig. 10 is a flowchart illustrating an example of a flow of a function restricting process executed by a control program of the analysis device 10 according to the first embodiment.

When execution of the function restricting process by the control program of the analysis device 10 is instructed, the control unit 6 reads and executes the control program stored in the storage unit 15.

In Step S101 of Fig. 10, the control unit 6 determines whether or not the operation unit 20 is detached from the main body 1. In a case in which it is determined that the operation unit 20 has not been detached (in the case of negative determination), the process proceeds to Step S102, and in a case in which it is determined that the operation unit 20 has been detached (in the case of positive determination), the process proceeds to Step S103.

In Step S102, the control unit 6 permits the use of all the functions of the analysis device 10, displays the permission on the operation unit 20, and ends the function restricting process by the control program.

On the other hand, in Step S103, the control unit 6 acquires the distance D between the main body 1 and the operation unit 20 as illustrated in Fig. 8 described above as an example.

In Step S104, the control unit 6 determines whether the distance D acquired in Step S103 satisfies a condition of the distance D < the threshold Th1. In a case in which it is determined that the distance D < the threshold Th1 (in the case of positive determination), the process proceeds to Step S105, and in a case in which it is determined that the condition of the distance D < the threshold Th1 is not satisfied, that is, the distance D ≥ the threshold Th1 (in the case of negative determination), the process proceeds to Step S106.

In Step S105, the control unit 6 restricts some functions of the drive system of the analysis device 10, displays the restriction on the operation unit 20, and ends the function restricting process by the control program.

On the other hand, in Step S106, the control unit 6 restricts the use of all the functions of the analysis device 10, displays the restriction on the operation unit 20, and ends the function restricting process by the control program.

As described above, according to the embodiment, it is possible to restrict a function of a device depending on a distance between an analysis device main body and an operation unit that is attachable to and detachable from the main body while deterioration of user convenience is suppressed. Consequently, the safety of the user who works by directly touching the device is ensured. In addition, leakage of personal information on a patient or the like is suppressed, and the personal information is protected.

### [Second Embodiment]

In the first embodiment, a mode in which use of all the functions is permitted in a case in which the operation unit is attached to the main body has been described. In a second embodiment, a mode in which use of all the functions can be permitted even in a case in which the operation unit is detached from the main body will be described.

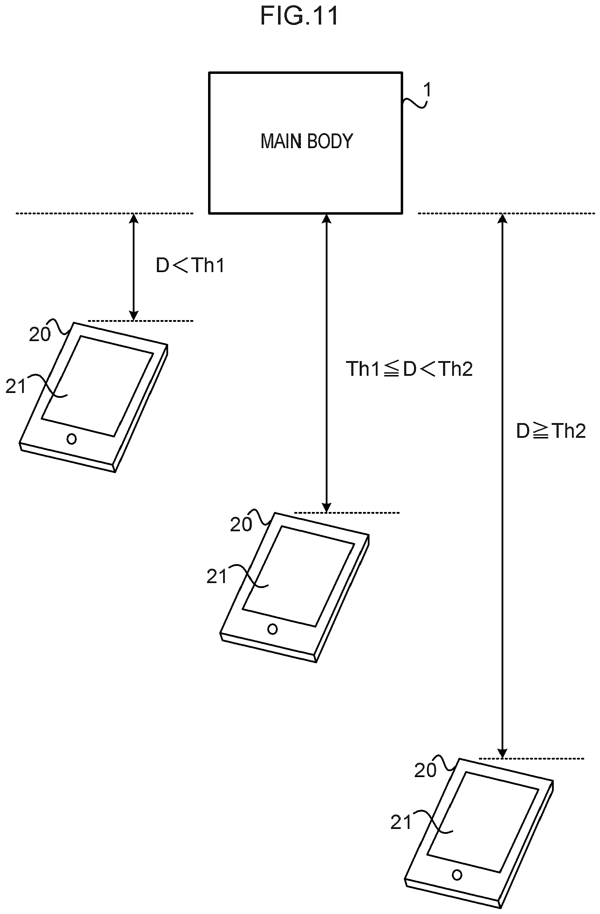

Fig. 11 is a diagram for explaining a functional restriction depending on a distance D between a main body 1 and an operation unit 20 according to the second embodiment.

As illustrated in Fig. 11, in a case in which the distance D between the main body 1 and the operation unit 20 is less than a first threshold Th1, a function restricting unit 6B according to the embodiment permits the use of all the functions. In a case in which the distance D is equal to or larger than the first threshold Th1 and less than a second threshold Th2, the function restricting unit 6B restricts at least some of the functions of the drive system. In a case in which the distance D is equal to or larger than the second threshold Th2, the function restricting unit 6B restricts the use of all the functions.

A threshold setting unit 6C displays a setting screen on the operation unit 20 and can set at least one of the first threshold Th1 or the second threshold Th2 according to a use environment of the user. For example, in a case in which the user who operates the operation unit 20 cannot see the main body 1 although the distance to the main body 1 is short, at least one of the first threshold Th1 or the second threshold Th2 can be set so that all the functions are limited even with a short distance.

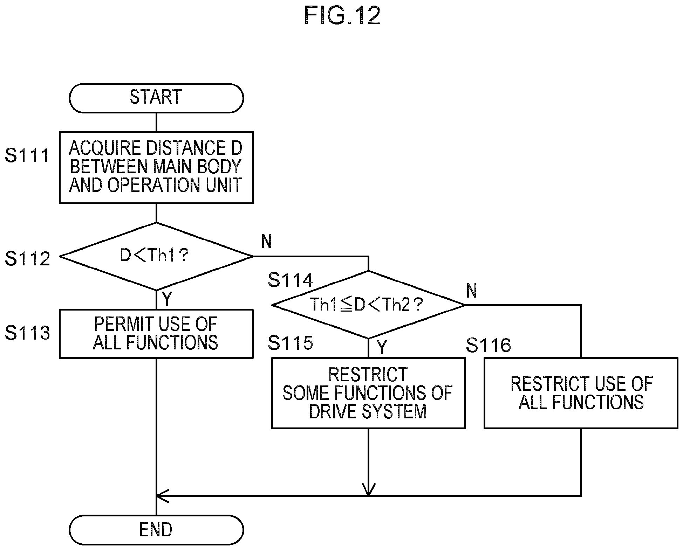

Next, an operation of an analysis device 10 according to the second embodiment will be described with reference to Fig. 12.

Fig. 12 is a flowchart illustrating an example of a flow of a function restricting process executed by a control program of the analysis device 10 according to the second embodiment.

When execution of the function restricting process by the control program of the analysis device 10 is instructed, the control unit 6 reads and executes the control program stored in the storage unit 15.

In Step S111 of Fig. 12, a control unit 6 acquires the distance D between the main body 1 and the operation unit 20 as illustrated in Fig. 11 described above as an example.

In Step S112, the control unit 6 determines whether the distance D acquired in Step S111 satisfies a condition of the distance D < the first threshold Th1. In a case in which it is determined that the distance D < the first threshold Th1 (in the case of positive determination), the process proceeds to Step S113, and in a case in which it is determined that the condition of the distance D < the threshold Th1 is not satisfied (in the case of negative determination), the process proceeds to Step S114.

In Step S113, the control unit 6 restricts the use of all the functions of the analysis device 10, displays the restriction on the operation unit 20, and ends the function restricting process by the control program.

On the other hand, in Step S114, the control unit 6 determines whether the distance D acquired in Step S111 satisfies a condition of the first threshold Th1 ≤ the distance D < the second threshold Th2. In a case in which it is determined that the first threshold Th1 ≤ the distance D < the second threshold Th2 (in the case of positive determination), the process proceeds to Step S115, and in a case in which it is determined that the condition of the first threshold Th1 ≤ the distance D < the second threshold Th2 is not satisfied, that is, the distance D ≥ the second threshold Th2 (in the case of negative determination), the process proceeds to Step S116.

In Step S 115, the control unit 6 restricts some functions of the drive system of the analysis device 10, displays the restriction on the operation unit 20, and ends the function restricting process by the control program.

On the other hand, in Step S 116, the control unit 6 restricts the use of all the functions of the analysis device 10, displays the restriction on the operation unit 20, and ends the function restricting process by the control program.

As described above, according to the embodiment, even in a case in which the operation unit is detached from the main body, use of all the functions can be permitted as long as the distance is relatively short.

### [Third Embodiment]

In a third embodiment, a mode in which an operation unit supports a plurality of analysis devices will be described.

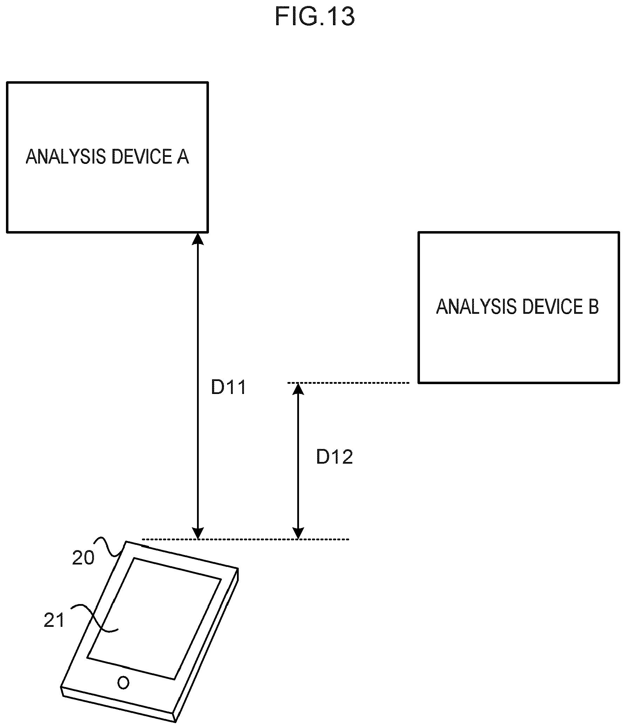

Fig. 13 is a diagram for describing a functional restriction in a case in which an operation unit 20 according to the third embodiment supports a plurality of analysis devices A and B.

As illustrated in Fig. 13, in a case in which the operation unit 20 supports the plurality of analysis devices A and B, a function restricting unit 6B according to the embodiment identifies an analysis device (for example, the analysis device B) having the shortest distance, of the plurality of analysis devices A and B, and restricts a function of the analysis device B depending on two or more levels of distance between a main body 1 of the identified analysis device B and the operation unit 20. Moreover, the number of the plurality of analysis devices may be three or more. In addition, the plurality of analysis devices A and B may be the same type of analysis device or different types of analysis devices.

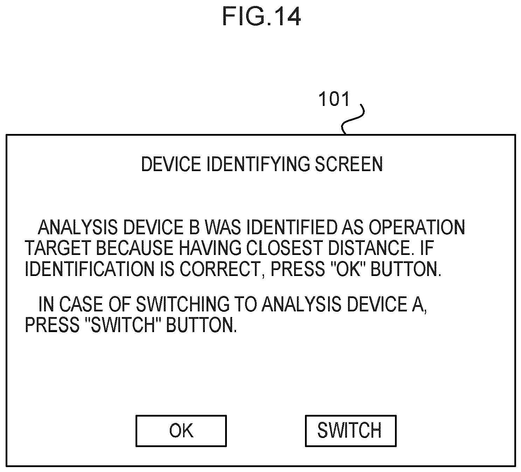

Fig. 14 is a front view illustrating an example of a device identifying screen 101 according to the third embodiment.

According to the device identifying screen 101 illustrated in Fig. 14, an "OK" button may be selected in a case in which the analysis device B identified by the function restricting unit 6B is an operation target, and a "switch" button may be selected in a case in which switching to the analysis device A is performed.

As described above, according to the embodiment, even in a case in which the operation unit supports a plurality of analysis devices, the analysis device that is the operation target can be identified, and the function of the identified analysis device can be limited depending on the distance.

The analysis device according to the embodiment has been described above as an example. The embodiment may be realized in forms of programs for causing a computer to execute functions of units included in the analysis device. The embodiment may be in a form of a non-transitory computer-readable storage medium storing these programs.

Additionally, the configuration of the analysis device described in the above embodiment is an example and may be changed according to a situation without departing from the gist.

In addition, the flow of processing of the program described in the above embodiment is also an example, and redundant steps may be removed, new steps may be added, or a processing order may be changed within a scope without departing from the gist.

In addition, in the above-described embodiment, a case in which the processing according to the embodiment is realized by a software configuration by using a computer by executing a program has been described, but the disclosure is not limited thereto. The embodiment may be realized by, for example, a hardware configuration or a combination of the hardware configuration and a software configuration.

Regarding the above embodiments, the following is further disclosed.

An analysis device according to a first aspect, includes a main body having a measurement unit that measures a component in a sample and an operation unit that is attachable to and detachable from the main body, the analysis device including: a function restricting unit that restricts a function of at least one of the main body or the operation unit depending on two or more levels of distance between the main body and the operation unit.

The analysis device according to a second aspect has a configuration in which, in the analysis device according to the first aspect, the function restricting unit permits use of all functions in a case in which the operation unit is attached to the main body, restricts at least some functions of the drive system in a case in which the operation unit is detached from the main body and the distance is smaller than a threshold, and restricts use of all the functions in a case in which the distance is equal to or larger than the threshold.

The analysis device according to a third aspect has a configuration in which, in the analysis device according to the second aspect, the function restricting unit restricts at least some functions of a drive system required for measurement of a component in the sample in a case in which the operation unit is detached from the main body and the distance is less than the threshold.

The analysis device according to a fourth aspect has a configuration in which, in the analysis device according to the first aspect, the function restricting unit permits use of all functions in a case in which the distance is less than a first threshold, restricts at least some functions of a drive system in a case in which the distance is equal to or larger than the first threshold and less than a second threshold, and restricts use of all functions in a case in which the distance is equal to or larger than the second threshold.

The analysis device according to a fifth aspect further includes, in the analysis device according to the fourth aspect, a threshold setting unit that enables a user to set at least one of the first threshold or the second threshold.

The analysis device according to a sixth aspect further includes, in the analysis device according to any one of the first to fifth aspects, a distance determining unit that determines the distance on the basis of the signal strength when communication is performed between the main body and the operation unit.

An analysis device according to a seventh aspect has a configuration in which, in the analysis device according to any one of the first to sixth aspects, in a case in which the operation unit supports a plurality of analysis devices, the function restricting unit identifies an analysis device having a shortest distance, of the plurality of analysis devices, and restricts the function depending on two or more levels of distance between the main body of the identified analysis device and the operation unit.

The analysis device according to an eighth aspect has a configuration in which, in the analysis device according to any one of the first to seventh aspects, the function restricting unit changes a function to be restricted on the basis of identification information of a user who logs in the analysis device.

The analysis device according to a ninth aspect further includes, in the analysis device according to any one of the first to eighth aspects, a detection unit that detects a person around the main body, in which the function restricting unit changes a function to be restricted in a case in which a person is not detected around the main body by the detection unit.

The analysis device according to a tenth aspect has a configuration in which, in the analysis device according to any one of the first to ninth aspects, the sample includes a biological sample.

A method for restricting a function according to an eleventh aspect is executed by an analysis device including a main body having a measurement unit that measures a component in a sample and an operation unit that is attachable to and detachable from the main body, the method including: restricting a function of at least one of the main body or the operation unit depending on two or more levels of distance between the main body and the operation unit.

A function restricting program according to a twelfth aspect of an analysis device including a main body having a measurement unit that measures a component in a sample and an operation unit that is attachable to and detachable from the main body is executable by a computer to perform a process restricting a function of at least one of the main body or the operation unit depending on two or more levels of distance between the main body and the operation unit.

## Claims

1. An analysis device (10), comprising:
a main body (1) having a measurement unit (7A) that measures a component in a sample and an operation unit (20) that is attachable to and detachable from the main body (1); and
a function restricting unit (6B) that restricts a function of at least one of the main body (1) or the operation unit (20) depending on two or more levels of distance between the main body (1) and the operation unit (20).

2. The analysis device (10) according to claim 1, wherein the function restricting unit (6B) permits use of all functions in a case in which the operation unit (20) is attached to the main body (1), restricts at least some functions of a drive system in a case in which the operation unit (20) is detached from the main body (1) and the distance is less than a threshold, and restricts use of all functions in a case in which the distance is equal to or larger than the threshold.

3. The analysis device (10) according to claim 2, wherein the function restricting unit (6B) restricts at least some functions of a drive system required for measurement of a component in the sample in a case in which the operation unit (20) is detached from the main body (1) and the distance is less than the threshold.

4. The analysis device (10) according to claim 1, wherein the function restricting unit (6B) permits use of all functions in a case in which the distance is less than a first threshold, restricts at least some functions of a drive system in a case in which the distance is equal to or larger than the first threshold and less than a second threshold, and restricts use of all functions in a case in which the distance is equal to or larger than the second threshold.

5. The analysis device (10) according to claim 4, further comprising:
a threshold setting unit (6C) that enables a user to set at least one of the first threshold or the second threshold.

6. The analysis device (10) according to claim 1, further comprising:
a distance determining unit (6A) that determines the distance based on a signal strength when communication is performed between the main body (1) and the operation unit (20).

7. The analysis device (10) according to any one of claims 1 to 6, wherein, in a case in which the operation unit (20) supports a plurality of analysis devices, the function restricting unit (6B) identifies an analysis device having a shortest distance, of the plurality of analysis devices, and restricts the function depending on two or more levels of distance between a main body (1) of the identified analysis device and the operation unit (20).

8. The analysis device (10) according to any one of claims 1 to 7, wherein the function restricting unit (6B) changes a function to be restricted based on identification information of a user who is logged in to the analysis device.

9. The analysis device (10) according to any one of claims 1 to 8, further comprising:
a detection unit (6D) that detects a person around the main body (1),
wherein the function restricting unit (6B) changes a function to be restricted in a case in which a person is not detected around the main body (1) by the detection unit (6D).

10. The analysis device (10) according to any one of claims 1 to 9, wherein the sample includes a biological sample.

11. A method for restricting a function by an analysis device (10) including a main body (1) having a measurement unit (7A) that measures a component in a sample and an operation unit (20) that is attachable to and detachable from the main body, the method comprising:
limiting a function of at least one of the main body (1) or the operation unit (20) depending on two or more levels of distance between the main body (1) and the operation unit (20), by the analysis device.

12. A non-transitory storage medium storing a function restricting program for an analysis device (10) including a main body (1) having a measurement unit (7A) that measures a component in a sample and an operation unit (20) that is attachable to and detachable from the main body, the function restriction program being executable by a computer to perform
a process including restricting a function of at least one of the main body (1) or the operation unit (20) depending on two or more levels of distance between the main body (1) and the operation unit (20).
